(19) Europäisches Patentamt
European Patent Office
Office européen des brevets

(11) Veröffentlichungsnummer: **0 015 502**
**A2**

# (12) EUROPÄISCHE PATENTANMELDUNG

(21) Anmeldenummer: **80100980.4**

(22) Anmeldetag: **28.02.80**

(51) Int. Cl.³: **C 07 D 249/08,** C 07 D 233/60, C 07 D 405/12, A 01 N 43/64, A 01 N 43/50 // (C07C69/653, 69/732)

(30) Priorität: **03.03.79 DE 2908323**

(43) Veröffentlichungstag der Anmeldung: **17.09.80 Patentblatt 80/19**

(84) Benannte Vertragsstaaten: **AT BE CH DE FR GB IT NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Ertel, Hartmut, Dr., Johann-Strauss-Strasse 24, D-6233 Kelkheim (Taunus) (DE)**
Erfinder: **Heubach, Günther, Dr., Luisenstrasse 15, D-6233 Kelkheim (Taunus) (DE)**
Erfinder: **Kocur, Jean, Dr., Pommernweg 14, D-6238 Hofheim am Taunus (DE)**
Erfinder: **Sachse, Burkhard, Dr., An der Ziegelei 30, D-6233 Kelkheim (Taunus) (DE)**

(54) **Azolsubstituierte Atropasäurederivate, Verfahren zu ihrer Herstellung und ihre Verwendung als Pflanzenschutzmittel.**

(57) Verbindungen der Formel I,

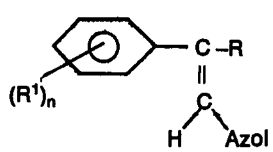

(I)

worin
$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,
n 0, 1, 2 oder 3,

$R^2$ eine $R^3\,X-\overset{\overset{O}{\|}}{C}$ -Gruppe, in der X Sauerstoff oder Schwefel, $R^3$ $C_{1-8}$-Alkyl, das gegebenenfalls substituiert ist, $C_{2-6}$-Alkenyl, $C_{2-6}$-Alkinyl, $C_{3-8}$-Cycloalkyl, das gegebenenfalls substituiert ist, Phenyl, das gegebenenfalls substituiert ist, Benzyl, das gegebenenfalls substituiert ist, oder einen den Epoxiring enthaltenden Alkylrest mit bis zu 4 C-Atomen bedeutet, oder

$R^2$ für –CN oder eine 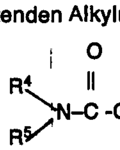 -Gruppe, in der für $R^4$ Wasserstoff oder $C_{1-4}$-Alkyl und für $R^5$ $C_{1-4}$-Alkyl oder Phenyl, das gegebenenfalls substituiert ist, oder $R^4$ und $R^5$ gegebenenfalls gemeinsam mit dem Amid-N-atom einen heterocyclischen Ring bilden, steht, und

Azol 1,2,4-Triazol-1-yl 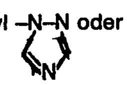 oder

1,2,4-Triazol-4-yl 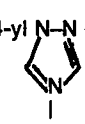 oder

Imidazol-1-yl 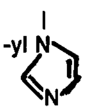

bedeuten, wobei die Verbindungen der Formel I auch in Form ihrer Salze mit anorganischen oder organischen Säuren oder als Metallkomplexverbindungen vorliegen können, ferner ein Verfahren zu ihrer Herstellung und ihre Verwendung als Schädlingsbekämpfungsmittel.

EP 0 015 502 A2

0015502



HOECHST AKTIENGESELLSCHAFT HOE 79/F 048 Dr.GM/Lo

# Azolsubstituierte Atropasäurederivate, Verfahren zu ihrer Herstellung und ihre Verwendung als Pflanzenschutzmittel

Gegenstand der vorliegenden Erfindung sind neue, azolsubstituierte Atropasäurederivate, ihre Herstellung und ihre Verwendung als Schädlingsbekämpfungsmittel im Pflanzenschutz.

Die erfindungsgemäßen Verbindungen besitzen die allgemeine Formel I,

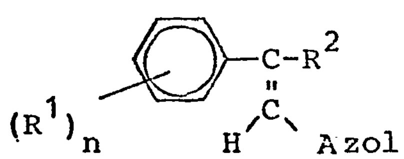

(I)

worin

$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,

n 0, 1, 2 oder 3,

$R^2$ eine $R^3X-\overset{O}{\overset{\|}{C}}$-Gruppe, in der

X Sauerstoff oder Schwefel,

$R^3$ $C_{1-8}$-Alkyl, das gegebenenfalls durch Halogen, $C_{1-3}$-Alkoxyreste oder durch Alkoxycarbonylreste mit bis zu 5 C-Atomen oder durch Dialkylaminoreste mit bis zu 8 C-Atomen substituiert ist, $C_{2-6}$-Alkenyl, $C_{2-6}$-Alkinyl, $C_{3-8}$-Cycloalkyl, das gegebenenfalls durch $C_{1-4}$-Alkylreste substituiert ist, Phenyl, das gegebenenfalls durch Halogenatome, $C_{1-3}$-Alkylreste, $C_{1-3}$-Alkoxyreste oder die $CF_3$-Gruppe substituiert ist, Benzyl, das gegebenenfalls durch Halogen substituiert ist, oder einen den Epoxiring enthaltenden Alkylrest mit bis zu 4 C-Atomen bedeutet, oder

$R^2$ für -CN oder eine 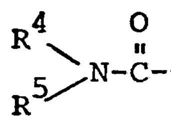-Gruppe, in der für $R^4$ Wasserstoff oder $C_{1-4}$-Alkyl und für $R^5$ $C_{1-4}$-Alkyl oder Phenyl, das gegebenenfalls durch Halogenatome, $CF_3$-Reste, $C_{1-4}$-Alkyl- oder $C_{1-4}$-Alkoxyreste oder durch den Phenoxy- oder Halogenphenoxyrest substituiert ist, oder $R^4$ und $R^5$ gegebenenfalls gemeinsam mit dem Amid-N-atom einen heterocyclischen Ring bilden, steht, und

Azol 1,2,4-Triazol-1-yl 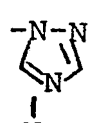 oder 1,2,4-Triazol-4-yl 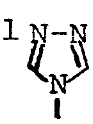

oder Imidazol-1-yl 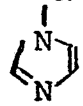

bedeuten.

Die Verbindungen der Formel I können auch in Form ihrer Salze mit anorganischen oder organischen Säuren oder als Metallkomplexverbindungen, vorzugsweise Kupfer- oder Zinkkomplexverbindungen vorliegen, wobei die letzteren in üblicher Weise, z.B. aus $CuCl_2$ oder $ZnCl_2$ und Verbindungen der Formel I erhalten werden können.

Bevorzugt bedeuten in Formel I $R^1$ Halogen, insbesondere Chlor, n 0 oder 1, X Sauerstoff, und Azol den 1,2,4-Triazol-1-yl-Rest.

Die Verbindungen der Formel I fallen im allgemeinen als E/Z-Isomerengemische an, die nach bekannten Verfahren in die einzelnen Isomeren getrennt werden können.

Die Verbindungen der Formel I können dadurch hergestellt werden, daß man Verbindungen der allgemeinen Formel II,

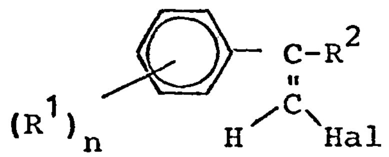

(II)

in der $R^1$, $R^2$ und n die Bedeutungen wie in Formel I haben und für Hal Halogen, vorzugsweise Chlor oder Brom, insbesondere Chlor steht, bei höheren Temperaturen, vorzugsweise bei 60 bis 120°C in Gegenwart eines Halogenwasserstoff bindenden Mittels, vorzugsweise mindestens einer stöchiometrischen oder überschüssigen Menge dieses Mittels, beispielsweise einer basischen Verbindung , wie z.B. Kaliumcarbonat, Natriumcarbonat, Triethylamin oder N,N-Dimethylanilin, mit mindestens einem Mol 1,2,4-Triazol oder Imidazol pro Mol Verbindung der Formel II umsetzt.

Die Umsetzung führt man vorteilhaft in inerten organischen Lösungsmitteln, wie z.B. Aceton, Acetonitril, Dimethylformamid oder Xylol durch.

Die zum Teil neuen Vorprodukte der Formel II können z.B. gemäß Reaktionsschema 1 bzw. 2 auf folgendem Weg erhalten werden:

Reaktionsschema 1

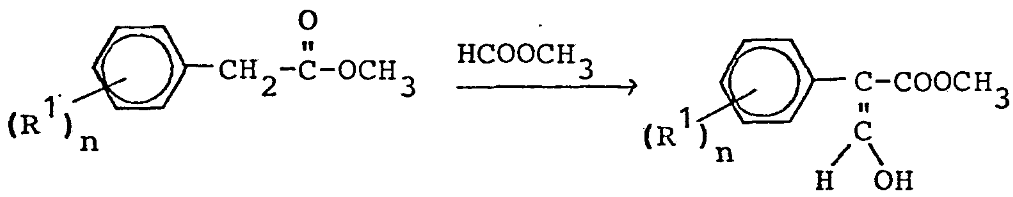


(III)

Dieser Reaktionstyp ist beschrieben in Liebigs Annalen der Chemie 413 (1917) S. 206.

Gemäß dem Reaktionsschema 2 erhält man durch Chlorieren der Verbindungen der Formel III Ester der Formel II a. Die mit schlechten Ausbeuten verlaufende Chlorierung mit $PCl_5$ ist für n = O beschrieben in Berichte dtsch. Chem. Ges. 51 (1918) S. 1368. Die mit wesentlich besseren Ausbeuten verlaufende Chlorierung mit $POCl_3$ in Dimethylformamid (DMF) ist noch nicht beschrieben. Anstelle von $POCl_3$ oder $PCl_5$ lassen sich z.B. auch Thionylchlorid, Phosphortrichlorid oder Phosgen verwenden.

Reaktionsschema 2

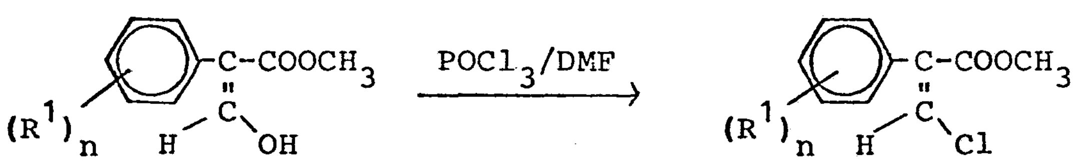


(III) (II a)

Höhere Ester lassen sich aus den Methylestern der Formel II a nach bekannten Methoden durch Umesterung herstellen.

Ein weiterer Syntheseweg für höhere Ester (II b) bzw. für Amide (II c) ist in dem Reaktionsschema 3 skizziert.

0015502

Reaktionsschema 3

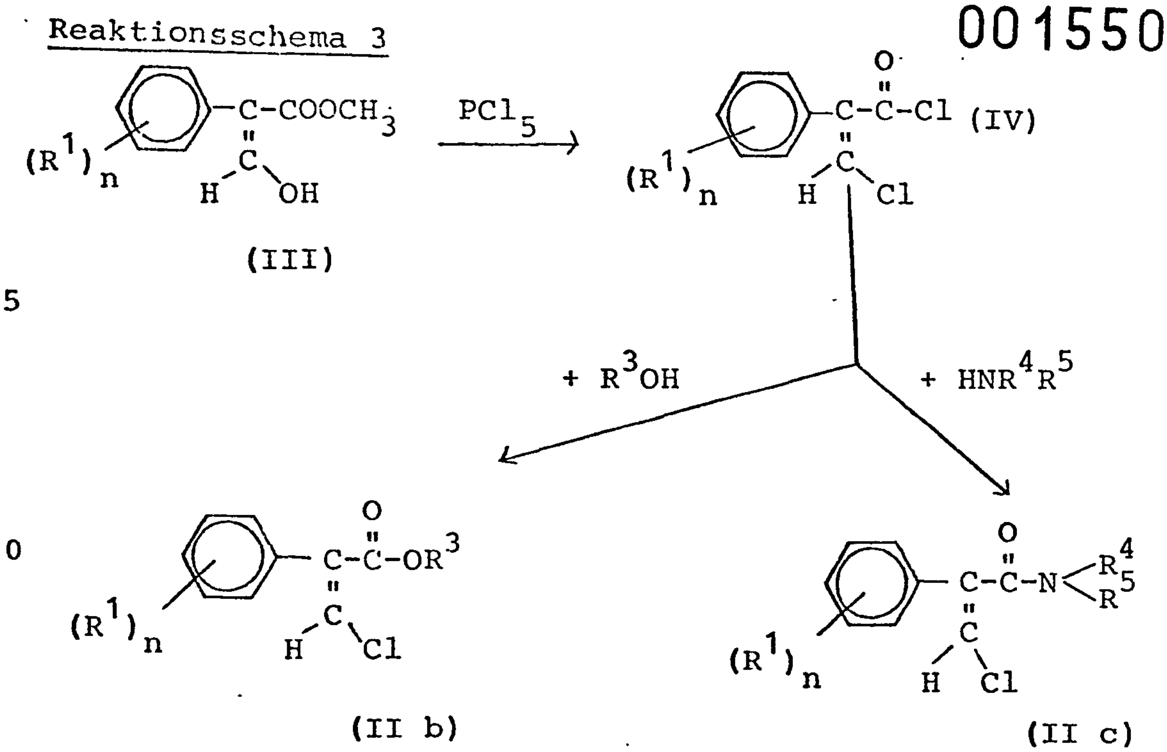


(III) (IV) (II b) (II c)

Die Chlorierungsreaktion III ⟶ IV ist für n = O beschrieben in Berichte dtsch. chem. Ges. 51 (1918) S. 1368.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt z.B. neben Piricularia oryzae und verschiedenen Rostarten vor allem Echte Mehltaupilze im Obst-, Gemüse- Getreide- und Zierpflanzen-Anbau. Besonders hervorzugeben ist die ausgezeichnete Wirkung der Verbindungen gegen Mehltauarten, die gegen Benzimidazolderivate (z.B. Benomyl, Carbendazim) resistent sind.

Die Verbindungen der Formel I eigenen sich auch für den Einsatz im technischen Bereich, beispielsweise in Holzschutzmitteln, auf dem Anstrichfarbensektor, als Konservierungsmittel, z.B. in Kühlschmiermitteln für die Metallbearbeitung.

Für die Anwendung im Pflanzenschutz können die fungiziden Verbindungen der Formel I in üblicher Weise als Stäube Spritzpulver, Beizmittel, Dispersionen, Lösungen oder Emulsionskonzentrate formuliert werden. Der Gehalt an Wirkstoffen der Formel I liegt im allgemeinen zwischen 2 und 95 Gew.%, vorzugsweise bei 10 -90 Gew.%. Daneben enthalten die genannten Wirkstoff-Formulierungen ggf. die jeweils üblichen Haft-, Netz-, Dispergier-, Emulgier -, Penetrations-, Lösungsmittel-, Füll- und Trägerstoffe. Auch Mischungen oder Mischformulierungen mit anderen pestiziden Wirkstoffen, wie z.B. Insektiziden, Akariziden, Herbiziden oder Fungiziden sowie von Düngemitteln bzw. von Wachstumsregulatoren sind ggf. möglich.

Die Erfindung wird durch die nachfolgenden Beispiele erläutert.

A. Vorprodukte für die Herstellungsbeispiele

1) 2-(4-Chlorphenyl)-3-chloracrylsäure-methylester

Zu einer aus 65,5 g (2,85 g-Atom) Natrium und 800 ml absolutem Toluol hergestellten Natriumsuspension läßt man ein Gemisch aus 504 g (2,73 Mol) p-Chlorphenylessigsäuremethylester, 164 g (2,73 Mol) Ameisensäuremethylester und 30 g Methanol so zulaufen, daß die Temperatur nicht über 40°C steigt. Nach beendetem Zulauf rührt man 4 Stunden bei 40°C und 12 Stunden bei Raumtemperatur. Das ausgefallene Natriumsalz des 2-(4-Chlorphenyl)-3-hydroxyacrylsäuremethylesters wird abgesaugt, mit 200 ml Toluol nachgewaschen, in 3 l Wasser eingetragen und mit verdünnter Schwefelsäure angesäuert (pH 2). Der 2-(4-Chlorphenyl)-3-hydroxyacrylsäuremethylester wird mit Methylenchlorid extrahiert, der Extrakt getrocknet und eingeengt. Man erhält 516 g eines semikristallinen Produktes, das ohne weitere Reinigung in 600 ml absolutem Dimethylformamid gelöst wird. Nach Zutropfen von 430 g (2,8 Mol) Phosphoroxychlorid bei 25°C läßt man 1 Std. nachrühren und erwärmt dann 1 Std. auf 110°C. Nach dem Abkühlen gießt man auf 4 l Eiswasser, extrahiert mit Methylenchlorid, trocknet den Extrakt, zieht das Lösungsmittel ab und destilliert den Rückstand. Man erhält 406 g (64,4 % d. Theorie) 2-(4-Chlorphenyl)-3-chlor-acrylsäuremethylester,

$$Kp_{1,8mbar}\ 121°C;\ n_D^{22}\ 1,5615.$$

2) 2-(4-Chlorphenyl)-3-chlor-acrylsäureisopropylester

115 g (0,5 Mol) 2-(4-Chlorphenyl)-3-chlor-acrylsäuremethylester werden mit 108 g Isopropanol und 5 g Zirkon(IV)acetylacetonat zum Rückfluß erhitzt. Innerhalb von 8 Stunden werden 300 ml Alkohol abdestilliert und gleichzeitig durch frisches Isopropanol ersetzt. Danach engt man das Reaktionsgemisch im Rotavapor ein und destilliert den Rückstand.

Man erhält 78 g (60,2 % d.Theorie) 2-(4-Chlorphenyl)-3-chlor-acrylsäureisopropylester,

$Kp_{0,1mbar}$ 92°C; $n_D^{22}$ 1,5395.

3) 2-(4-Chlorphenyl)-3-chlor-acrylsäureisobutylester

115 g (0,5 Mol) 2-(4-Chlorphenyl)-3-chlor-acrylsäuremethylester werden mit 153 g Isobutanol und 4 g p-Toluolsulfonsäure 14 Stunden unter Rückfluß gekocht. Während dieser Zeit werden 5 mal 100 ml Alkohol abdestilliert und gleichzeitig durch frischen ersetzt. Danach läßt man das Reaktionsgemisch abkühlen, zieht den überschüssigen Alkohol ab, nimmt den Rückstand in Methylenchlorid auf, wäscht die Lösung mit Wasser, trocknet sie, engt ein und destilliert.

Man erhält 82 g (60% d. Theorie) 2-(4-Chlorphenyl)-3-chlor-acrylsäureisobutylester

$Kp_{0,3mbar}$ 115 - 118°C; $n_D^{22}$ 1,5350.

4) 2-Phenyl-3-chlor-acrylsäureäthylester

Unter Rühren werden bei 40 - 45°C zu einer Mischung aus 140,8 g (0,733 Mol) 3-Hydroxy-2-phenyl-acrylsäureäthylester und 53,5 g (0,733 Mol) Dimethylformamid 112,3 g (0,733 mol) Phosphoroxychlorid zugetropft. Danach wird 90 Minuten bei 105°C nachgerührt. Nach dem Abkühlen wird das Gemisch in Eiswasser eingerührt und das abgeschiedene Öl mit Methylenchlorid aufgenommen. Nach dem Neutralwaschen der Methylenchloridlösung wird diese getrocknet und destilliert.

Es werden 133,5 g 2-Phenyl-3-chlor-acrylsäureäthylester erhalten. Ausbeute: 86% d. Theorie.

$Kp_{0,3-0,4mbar}$ 76 - 78°C.

5) 3-Hydroxy-2-phenyl-acrylsäure-äthylester

24 g Natrium (1,04 g-Atom) werden in 500 ml Toluol in fein verteilter Form suspendiert. Bei 20 - 22°C wird ein Gemisch aus 164,1 g (1,0 Mol) Phenylessigsäureäthylester und 74,1 g (1,0 Mol) Ameisensäureäthylester unter Rühren und Eiswasserkühlung innerhalb von 1 Stunde zugetropft und das Gemisch 5 Stunden nachgerührt. Nach 15 Stunden Stehen wird nochmals 1 Stunde lang auf 45°C erwärmt.

Das Reaktiongemisch wird vorsichtig mit 1 l Eiswasser versetzt. Nach Phasentrennung wird die wäßrige Phase aufgearbeitet unter Ansäuern mit Essigsäure, Ausschütteln mit Methylenchlorid, Trocknen des Methylenchloridextraktes und Destillieren. Es werden 140,8 g 3-Hydroxy-2-phenyl-acrylsäure-äthylester erhalten.

$Kp_{0,4-0,5\ mbar}$ 78 - 79°C; Ausbeute 73,3% d.Th.

B. Herstellungsbeispiele

Beispiel 1

2-(4-Chlorphenyl)-3-(1,2,4-triazol-1-yl)-acrylsäure-isobutylester

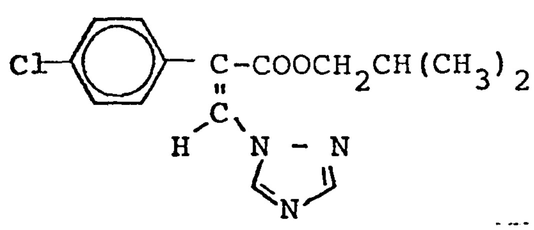

50 g (0,183 Mol) 2-(4-Chlorphenyl)-3-chlor-acrylsäure-isobutylester werden in 20 ml wasserfreiem Dimethylformamid gelöst und bei 25°C zu einer Suspension von 11,36 g (0,164 Mol) 1,2,4-Triazol und 27,6 g (0,2 Mol) Kaliumcarbonat in 180 ml Dimethylformamid getropft. Man rührt 3 Stunden bei 80°C, gibt 1,26 g (0,018 Mol) 1,2,4-Triazol zu und rührt weitere 2 Stunden bei 80°C. Nach Abkühlen wird

filtriert und das Lösungsmittel unter Vakuum abgezogen. Es verbleiben 59,7 g eines zähen Öls, das in Methylenchlorid aufgenommen, mit Wasser ausgeschüttelt, getrocknet, eingeengt und mit 60 ml Toluol/Hexan = 1 : 1 digeriert wird . Der ausgefallene 2-(4-Chlorphenyl)-3,3-bis-(1,2,4-triazol-1-yl)-propionsäureisobutylester wird abgesaugt (2,6 g) und die Mutterlauge unter Vakuum von dem Lösungsmittel befreit. Es verbleiben
46,4 g wachsartiger 2-(4-Chlorphenyl)-3-(1,2,4-triazol-1-yl)-acrylsäureisobutylester.
Ausbeute: 82,9% der Theorie

<u>Analyse:</u>
$C_{15}H_{16}ClN_3O_2$; (M = 305,7)

| | | | |
|---|---|---|---|
| Ber.: | C 58,92% | Gef.: | C 58,4% |
| | H 5,24% | | H 5,2% |
| | N 13,75% | | N 13,4% |

<u>Beispiel 2</u>

<u>2-(4-Chlorphenyl)-3-(1,2,4-triazol-1-yl)-acrylsäure-isopropylester</u>

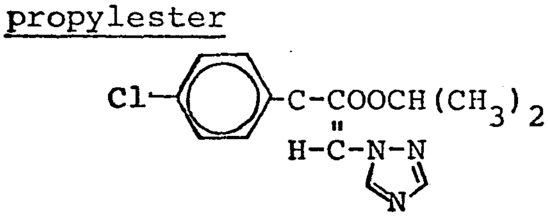

51 g (0,2 Mol) 2-(4-Chlorphenyl)-3-chloracrylsäureisopropylester werden in 30 ml wasserfreiem Dimethylformamid bei 25°C zu einer Suspension aus 12,42 g (0,18 Mol) 1,2,4-Triazol und 28,9 g (0,21 Mol) Kaliumcarbonat in 180 ml DMF getropft.

Man rührt 6 Stunden bei 70°C, gibt 1,38 g (0,02 Mol) 1,2,4-Triazol zu und rührt erneut 2 Stunden bei 70°C. Nach Abkühlen wird filtriert und das Lösungsmittel unter Vakuum abgezogen. Es verbleiben 72 g öliger Rückstand, der in Methylenchlorid aufgenommen, mit Wasser ausgeschüttelt, getrocknet und unter Vakuum von dem Lösungsmittel befreit wird.

Der ölige Rückstand verfestigt sich zu dem semikristallinen Isomerengemisch (44,4 g) aus 2-(4-Chlorphenyl)-3-(1,2,4-triazol-1-yl)-acrylsäureisopropylester.
Ausbeute: 76,1% der Theorie
$R_F$-Werte: 0,53 und 0,42 für das cis/trans (ca. 1:1)-Isomerengemisch (gemessen an Fertigplatten Kieselgel 60 $F_{254}$ (Merck), Laufmittel: Äthylacetat).

Analyse:
$C_{14}H_{14}ClN_3O_2$; (M = 291,7)

| | | | |
|---|---|---|---|
| Ber. | C 57,64% | Gef. | C 57,4% |
| | H 4,84% | | H 4,7% |
| | N 14,41% | | N 14,2% |

## Beispiel 3

2-Phenyl-3-(1,2,4-triazol-1-yl)-acrylsäureäthylester

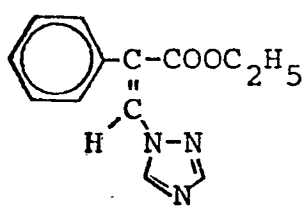

31,6 g (0,15 Mol) 3-Chlor-2-phenyl-acrylsäure-äthylester,
10,35 g (0,15 Mol) 1,2,4-Triazol und
27,6 g (0,20 Mol) Kaliumcarbonat werden in
100 ml Dimethylformamid 11 Stdn. bei 80°C gerührt. Dann wird vom Kaliumsalz abgesaugt und aus dem Filtrat das Dimethylformamid durch Vakuumdestillation weitgehend entfernt. Das Rückstandsöl wird in Methylenchlorid aufgenommen und mit Wasser gewaschen. Nach Trocknung der Lösung wird unter Vakuum das Lösungsmittel entfernt.
Es werden 32,9 g öliger 2-Phenyl-3-(1,2,4-triazol-1-yl)-acrylsäureäthylester erhalten, $n_D^{30}$ 1,5662.
Ausbeute: 90,1% der Theorie

Analyse:
$C_{13}H_{13}N_3O_2$ (M = 243,3)

| | | | |
|---|---|---|---|
| Ber.: | C 64,18% | Gef.: | C 63,9 % |
| | H 5,39 % | | H 5,5 % |
| | N17,27 % | | N 16,9 % |

Beispiele 4 - 94

In analoger Weise wie in Beispiel 1 beschrieben werden die Beispiele 4 bis 94 ausgeführt. In der Tabelle 1 sind die Reste $R^1$, $R^2$, A sowie n in der Formel Ia der nach den Beispielen 4 bis 94 aus den entsprechenden Verbindungen der Formel II hergestellten Verbindungen und deren Schmelzpunkte bzw. der Brechnungsindex aufgeführt.

0015502

Tabelle 1

Formel I a: 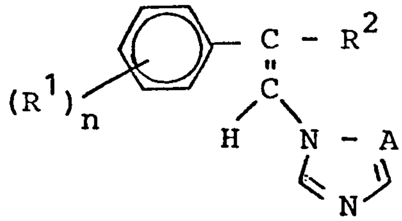

| Beispiel Nr. | $R^1$ | n | A | $R^2$ | Schmp. °C | Brechungs-index n |
|---|---|---|---|---|---|---|
| 4 | 4-Cl | 1 | -N= | $-\underset{\overset{\|}{O}}{C}-OC_2H_5$ | Sirup | |
| 5 | 4-Cl | 1 | -N= | $-\underset{\overset{\|}{O}}{C}-OCH_2CH_2CH_3$ | semikrist. | |
| 6 | 4-Cl | 1 | -N= | $-\underset{\overset{\|}{O}}{C}-OCH_3$ | 93-105 | |
| 7 | 4-Cl | 1 | -N= | $-\underset{\overset{\|}{O}}{C}-O(CH_2)_3CH_3$ | 57-68 | |
| 8 | 4-Cl | 1 | -N= | $-\underset{\overset{\|}{O}}{C}-O(CH_2)_4CH_3$ | Sirup | $n_D^{22}$ 1,5618 |
| 9 | 4-Cl | 1 | -N= | $-\underset{\overset{\|}{O}}{C}-O(CH_2)_5CH_3$ | Sirup | $n_D^{22}$ 1,5502 |
| 10 | 4-Cl | 1 | -N= | $-\underset{\overset{\|}{O}}{C}-O(CH_2)_2CH(CH_3)_2$ | Sirup | $n_D^{23}$ 1,5608 |
| 11 | 4-Cl | 1 | -N= | $-\underset{\overset{\|}{O}}{C}-O-\underset{\overset{\|}{CH_3}}{CH}-CH_2CH(CH_3)_2$ | semi-krist. | |
| 12 | 4-Cl | 1 | -N= | $-\underset{\overset{\|}{O}}{C}-O-\underset{\overset{\|}{CH_3}}{CH}(CH_2)_2CH_3$ | semikrist. | |
| 13 | 4-Cl | 1 | -N= | $-\underset{\overset{\|}{O}}{C}-O-$ 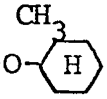 | semikrist. | |
| 14 | 4-Cl | 1 | -N= | $-\underset{\overset{\|}{O}}{C}-O-CH_2-$ 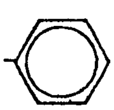 | Sirup | |
| 15 | | 0 | -N= | $-\underset{\overset{\|}{O}}{C}-OCH_3$ | Sirup | |
| 16 | | 0 | -N= | $-\underset{\overset{\|}{O}}{C}-O(CH_2)_2CH_3$ | Sirup | |
| 17 | | 0 | -N= | $-\underset{\overset{\|}{O}}{C}-OCH_2CH(CH_3)_2$ | semikrist. | |

| Beispiel Nr. | $R^1$ | n | A | $R^2$ | Schmp. °C | Brechungsindex n |
|---|---|---|---|---|---|---|
| 18 | | 0 | -N= | $-\underset{O}{\underset{\|}{C}}-OCH(CH_3)_2$ | öl | $n_D^{30}$ 1,5572 |
| 19 | | 0 | -N= | $-\underset{O}{\underset{\|}{C}}-O(CH_2)_3CH_3$ | öl | $n_D^{30}$ 1,5542 |
| 20 | | 0 | -N= | $-\underset{O}{\underset{\|}{C}}-O(CH_2)_4CH_3$ | öl | $n_D^{30}$ 1,5470 |
| 21 | | 0 | -N= | $-\underset{O}{\underset{\|}{C}}-O(CH_2)_2CH(CH_3)_2$ | öl | $n_D^{30}$ 1,5500 |
| 22 | | 0 | -N= | $-\underset{O}{\underset{\|}{C}}-O-CH_2\underset{CH_3}{CH}CH_2CH_3$ | öl | $n_D^{30}$ 1,5452 |
| 23 | | 0 | -N= | $-\underset{O}{\underset{\|}{CO}}-(CH_2)_5-CH_3$ | öl | $n_D^{30}$ 1,5402 |
| 24 | | 0 | -N= | $-\underset{O}{\underset{\|}{C}}OCH_2-$ 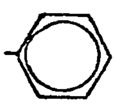 | öl | $n_D^{30}$ 1,5960 |
| 25 | | 0 | -N= | $-\underset{O}{\underset{\|}{C}}OCH_2CH=CH_2$ | öl | $n_D^{30}$ 1,5760 |
| 26 | | 0 | -N= | $-\underset{O}{\underset{\|}{C}}OCH(CH_2)_2CH_3$ ($CH_3$ an CH) | öl | $n_D^{30}$ 1,5500 |
| 27 | | 0 | -N= | -CN | 148,5 | |
| 28 | 4-Cl | 1 | -N= | -CN | 172-173 | |
| 29 | | 0 | -N= | $-\underset{O}{\underset{\|}{C}}-\underset{CH(CH_3)_2}{N}-$ 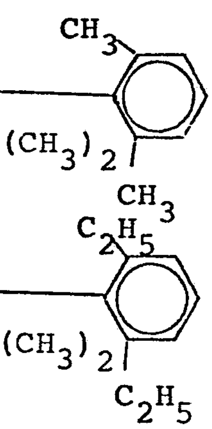 | Sirup | |
| 30 | | 0 | -N= | $-\underset{O}{\underset{\|}{C}}-\underset{CH(CH_3)_2}{N}-$ | öl | $n_D^{30}$ 1,5739 |
| 31 | | 0 | -N= | $-\underset{O}{\underset{\|}{C}}-\underset{CH(CH_3)_2}{N}-$ 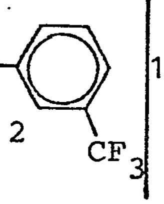 | 104-108 | |

| Beispiel Nr. | $R^1$ | n | A | $R^2$ | Schmp. °C | Brechungsindex n |
|---|---|---|---|---|---|---|
| 32 | | 0 | -N= | 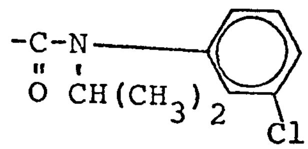 | 127-134 | |
| 33 | | 0 | -N= | 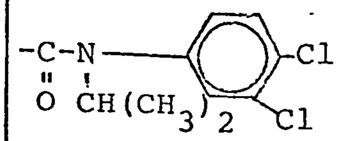 | öl | $n_D^{30}$ 1,5800 |
| 34 | | 0 | -N= | 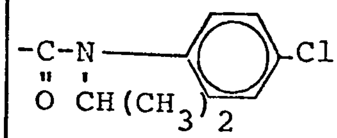 | 116-118 | |
| 35 | | 0 | -N= | 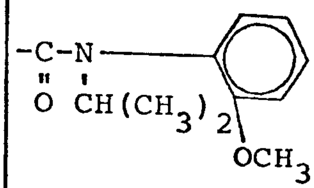 | öl | $n_D^{30}$ 1,5735 |
| 36 | | 0 | -N= | 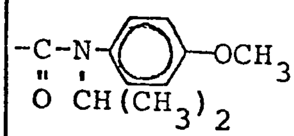 | 120-122 | |
| 37 | | 0 | -N= | 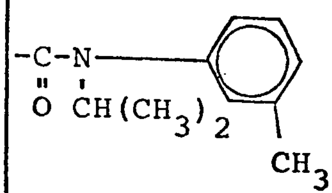 | 110-113 | |
| 38 | | 0 | -N= | 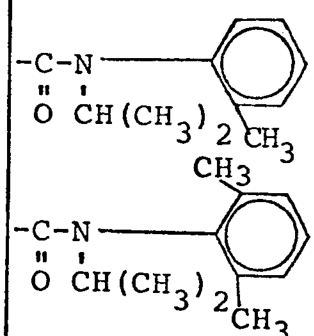 | semikrist. | |
| 39 | 4-Cl | 1 | -N= | | öl | $n_D^{30}$ 1,5691 |
| 40 | 4-Cl | 1 | -N= | 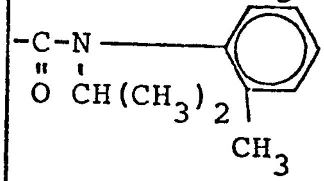 | öl | $n_D^{30}$ 1,5548 |
| 41 | 4-Cl | 1 | $-\underset{H}{C}=$ | $-\underset{O}{C}-O(CH_2)_5CH_3$ | Sirup | |

| Beispiel Nr. | $R^1$ | n | A | $R^2$ | Schmp. °C | Brechungsindex n |
|---|---|---|---|---|---|---|
| 42 | 4-Cl | 1 | -C=<br>H | 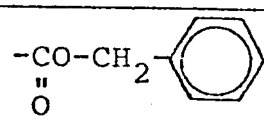 | Sirup | |
| 43 | 4-Cl | 1 | -C=<br>H | $-COCH_2CH_3$<br>O | 131,5 | |
| 44 | 4-Cl | 1 | -C=<br>H | $-COCH_3$<br>O | 152-153 | |
| 45 | | 0 | -C=<br>H | $-COCH_3$<br>O | 102-103 | |
| 46 | | 0 | -C=<br>H | 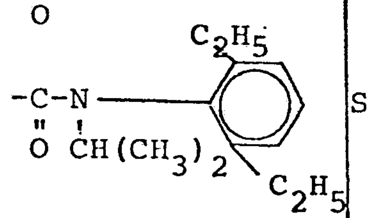 | Sirup | |
| 47 | | 0 | -C=<br>H | -CN | 131-132 | |
| 48 | | 0 | -C=<br>H | $-C-OCH(CH_3)_2$<br>O | 89-90 | |
| 49 | 4-Cl | 1 | -N= | $-C-OCH_2-CH=CH_2$<br>O | Sirup | |
| 50 | 4-Cl | 1 | -N= | $-C-N(C_2H_5)_2$<br>O | 113 | |
| 51 | 4-Cl | 1 | -N= | $-C-N(n-C_3H_7)_2$<br>O | 78 | |
| 52 | 4-Cl | 1 | -N= | 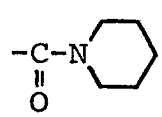 | 136 | |
| 53 | 4-Cl | 1 | -N= | 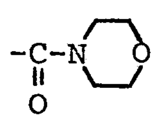 | 196 - 198 | |
| 54 | 4-Cl | 1 | -N= | 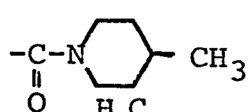 | 91 - 92 | |
| 55 | 4-Cl | 1 | -N= | 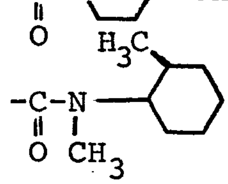 | 135 - 137 | |

| Beispiel Nr. | $R^1$ | n | A | $R^2$ | Schmp. °C | Brechungsindex n |
|---|---|---|---|---|---|---|
| 56 | 4-Cl | 1 | -N= | 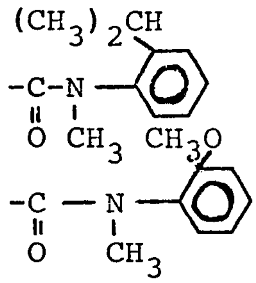 | glasartig | |
| 57 | 4-Cl | 1 | -N= | | 162 | |
| 58 | 4-Cl | 1 | -N= | 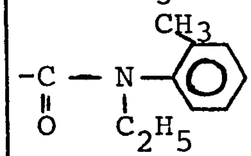 | 140 - 142 | |
| 59 | 4-Cl | 1 | -N= | 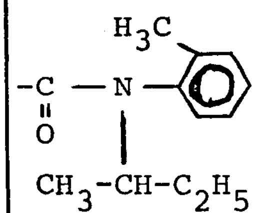 | 80 - 90 | |
| 60 | 4-Cl | 1 | -N= | 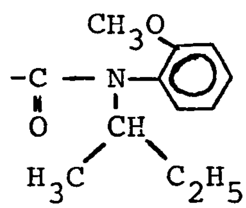 | 114 | |
| 61 | 4-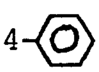 | 1 | -N= | 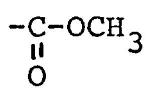 | 94 - 102 | |
| 62 | 4-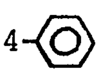 | 1 | -CH= | 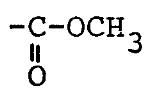 | 159 - 161 | |
| 63 | $3-OCF_2CF_2H$ | 1 | -N= | 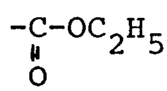 | Sirup | |
| 64 | $3-OCF_2CF_2H$ | 1 | -CH= | 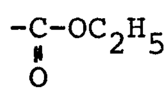 | semikrist. | |
| 65 | $3-CF_3$ | 1 | -N= | 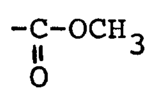 | 61 - 65 | |
| 66 | $3-CF_3$ | 1 | -N= | 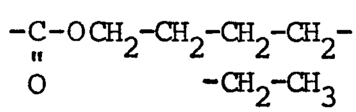 | | $n_D^{20}$: 1,5089 |
| 67 | | 0 | -N= | 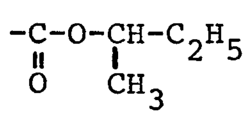 | Sirup | |
| 68 | | 0 | -N= | 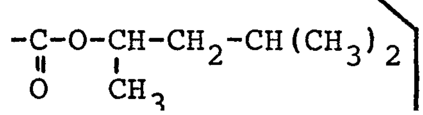 | Sirup | |

| Beispiel Nr. | $R^1$ | n | A | $R^2$ | Schmp. °C | Brechungsindex n |
|---|---|---|---|---|---|---|
| 69 | | 0 | -N= | $-\underset{\overset{\|}{O}}{C}-N(C_2H_5)_2$ | 100 - 101 | |
| 70 | | 0 | -N= | $-\underset{\overset{\|}{O}}{C}-N(CH_2C_2H_5)_2$ | 68 - 72 | |
| 71 | | 0 | -N= | 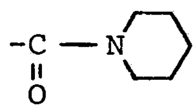 | 106 - 110 | |
| 72 | | 0 | -CH= | $-\underset{\overset{\|}{O}}{C}-NHC(CH_3)_3$ | 80 - 84 | |
| 73 | 2-Cl | 1 | -N= | $-\underset{\overset{\|}{O}}{C}-OCH_3$ | Sirup | |
| 74 | 2-Cl | 1 | -N= | $-\underset{\overset{\|}{O}}{C}-OC_2H_5$ | Sirup | $n_D^{30}$ 1,5724 |
| 75 | 2-Cl | 1 | -N= | 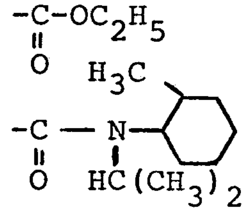 | Sirup | |
| 76 | 2-Cl | 1 | -N= | $-\underset{\overset{\|}{O}}{C}-N(CH_2C_2H_5)_2$ | Sirup | $n_D^{30}$ 1,5493 |
| 77 | 2-Cl | 1 | -N= | $-\underset{\overset{\|}{O}}{C}-N(C_2H_5)_2$ | Sirup | |
| 78 | 2-Cl | 1 | -CH= | -CN | Sirup | |
| 79 | 2-Cl | 1 | -CH= | $-\underset{\overset{\|}{O}}{C}-OCH_3$ | 80 - 81 | |
| 80 | 2-Cl | 1 | -CH= | $-\underset{\overset{\|}{O}}{C}-OC_2H_5$ | 97 - 99 | |
| 81 | 2-Cl | 1 | -CH= | $-\underset{\overset{\|}{O}}{C}-N(C_2H_5)_2$ | Sirup | |
| 82 | 2-Cl | 1 | -CH= | $-\underset{\overset{\|}{O}}{C}N(CH_3)_2$ | 87 - 89 | |

| Beispiel Nr. | $R^1$ | n | A | $R^2$ | Schmp. °C | Brechungsindex n |
|---|---|---|---|---|---|---|
| 83 | | 0 | -CH= | $-\underset{\underset{O}{\|}}{C}-OC_2H_5$ | 42 - 47 | |
| 84 | | 0 | -CH= | 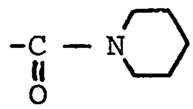 | 142 - 150 | |
| 85 | 3,4-$Cl_2$ | 2 | -N= | $-\underset{\underset{O}{\|}}{C}-N(CH_3)_2$ | 158 - 160 | |
| 86 | 3,4-$Cl_2$ | 2 | -N= | $-\underset{\underset{O}{\|}}{C}-N(C_2H_5)_2$ | 136 - 138 | |
| 87 | 3,4-$Cl_2$ | 2 | -N= | $-\underset{\underset{O}{\|}}{C}-N(CH_2C_2H_5)_2$ | Sirup | |
| 88 | 2,4-$Cl_2$ | 2 | -N= | $-\underset{\underset{O}{\|}}{C}-N(CH_2C_2H_5)_2$ | Sirup | |
| 89 | 2,4-$Cl_2$ | 2 | -N= | 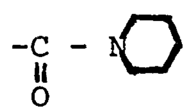 | Sirup | |
| 90 | 2,4-$Cl_2$ | 2 | -CH= | $-\underset{\underset{O}{\|}}{C}-N(C_2H_5)_2$ | Sirup | |
| 91 | 2,4-$Cl_2$ | 2 | -CH= | 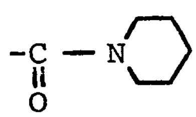 | 131 - 132 | |
| 92 | 4-$CH_3O$- | 1 | -N= | $-\underset{\underset{O}{\|}}{C}-OCH_3$ | 86 - 88 | |
| 93 | 4-$CH_3O$- | 1 | -CH= | $-\underset{\underset{O}{\|}}{C}-OCH_3$ | 92 - 96 | |
| 94 | 4-$CH_3O$- | 1 | -CH= | $-\underset{\underset{O}{\|}}{C}-OC_2H_5$ | 84 - 88 | |

0015502

Beispiele 95 - 105

Die organischen Liganden der Komplexverbindungen der Formel Ib der Beispiele 95 bis 105 werden zunächst analog dem Beispiel 1 hergestellt und anschließend mit den entsprechenden Metallchloriden Met.$Cl_2$ ($ZnCl_2$ bzw. $CuCl_2$) in üblicher Weise in die Komplexverbindungen der Formel Ib, wie sie in der Tabelle 2 wiedergegeben ist, übergeführt. In der Tabelle 2 sind die Reste $R^1$, $R^2$, A, n sowie das Metall (Met.) in der Formel Ib der nach den Beispielen 95 bis 105 hergestellten Metallkomplexverbindungen und deren Schmelzpunkte aufgeführt.

Tabelle 2

Formel Ib: 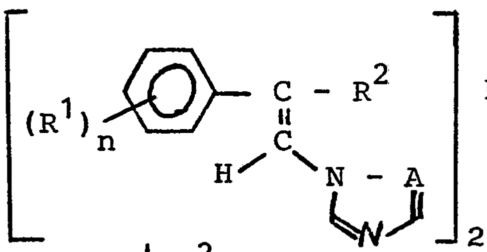 Met.$Cl_2$

| Beispiel Nr. | $R^1$ | n | A | $R^2$ | Met. | Schmp. (unter Zersetzung) |
|---|---|---|---|---|---|---|
| 95 | | 0 | -CH= | -CN | Cu | 233 |
| 96 | | 0 | -CH= | $-\underset{\overset{\|}{O}}{C}OC_2H_5$ | Cu | 139 - 140 |
| 97 | 2-Cl | 1 | -N= | $-\underset{\overset{\|}{O}}{C}-OC_2H_5$ | Zn | 151 |
| 98 | 2-Cl | 1 | -CH= | -CN | Zn | 200 - 201 |
| 99 | | 0 | -N= | $-\underset{\overset{\|}{O}}{C}-N(C_2H_5)_2$ | Cu | 207 - 208 |
| 100 | | 0 | -N= | $-\underset{\overset{\|}{O}}{C}-N(CH_2C_2H_5)_2$ | Cu | 187 |
| 101 | | 0 | -N= | $-\underset{\overset{\|}{O}}{C}-N(C_2H_5)_2$ | Zn | 230 |
| 102 | 2-Cl | 1 | -N= | 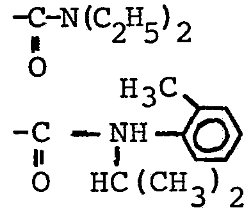 | Cu | 195 |

| Beispiel Nr. | $R^1$ | n | A | $R^2$ | Met. | Schmp. (unter Zersetzung) |
|---|---|---|---|---|---|---|
| 103 | | 0 | -N= | $-\underset{\underset{O}{\|}}{C}-NH-C(CH_3)_3$ | Cu | 247 |
| 104 | 4-Cl | 1 | -N= | $-COOCH_3$ | Cu | 242 |
| 105 | 4-Cl | 1 | -N= | $-COOC_2H_5$ | Cu | 187 |

## C. Formulierungsbeispiele

### Beispiel A:

Ein Stäubemittel wird erhalten, indem man

10 Gewichtsteile Wirkstoff
und 90 Gewichtsteile Talkum als Inertstoff

mischt und in einer Schlagmühle zerkleinert.

### Beispiel B:

Ein in Wasser leicht dispergierbares, benetzbares Pulver wird erhalten, indem man

25 Gewichtsteile Wirkstoff
64 Gewichtsteile kaolinhaltigen Quarz als Inertstoff
10 Gewichtsteile ligninsulfonsaures Kalium
und 1 Gewichtsteil oleoylmethyltaurinsaures Natrium als Netz- und Dispergiermittel

mischt und in einer Stiftmühle mahlt.

### Beispiel C:

Ein in Wasser leicht dispergierbares Dispersionskonzentrat wird erhalten, indem man

20 Gewichtsteile Wirkstoff mit
6 Gewichtsteilen Alkylphenolpolyglykoläther (Triton X 207)
3 Gewichtsteilen Isotridecanolpolyglykoläther (8 AeO)+)
und 71 Gewichtsteilen paraffinischem Mineralöl (Siedebereich z.B. ca. 255 bis über 377°C)

mischt und in einer Reibkugelmühle auf eine Feinheit von unter 5 Mikron vermahlt.

+) Anzahl AeO= Anzahl Äthylenoxydeinheiten im Polyglykoläthermolekül.

Beispiel D:

Ein emulgierbares Konzentrat wird erhalten aus

15 Gewichtsteilen Wirkstoff
75 Gewichtsteilen Cyclohexanon als Lösungsmittel
und 10 Gewichtsteilen oxäthyliertes Nonylphenol (10 AeO)+) als Emulgator.

+) Anzahl AeO= Anzahl Äthylenoxydeinheiten im Nonylphenolpolyglykoläthermolekül.

## D. Biologische Beispiele

In den folgenden Beispielen stehen die Buchstaben A, B, C für die nachstehend genannten handelsüblichen Vergleichsmittel:

A : Methyl-1-(butylcarbamoyl)-2-benzimidazolcarbamat (Benomyl)
B : N-Tridecyl-2,6-dimethyl-morpholin (Tridemorph)
C : 2-(sec. Butyl)-4,6-dinitro-phenyl-(3-methyl-2-butenoat) (Binapacryl)

Beispiel I

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90 - 95% aufgestellt. 3 Tage nach Inokulation werden die Pflanzen mit den in Tabelle I aufgeführten Verbindungen in den Wirkstoffkonzentrationen von 500, 250, 125, 60, 30 und 15 mg/Liter Spritzbrühe tropfnaß gespritzt. Als Vergleich wird das Vergleichsmittel B in analoger Weise eingesetzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100% Befall). Das Ergebnis ist in der Tabelle I zusammengefaßt.

Tabelle I

| Verbindung gemäß Beispiel Nr. | mit Weizenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 15 | 0 | 0 | 3-5 | 5-10 | 15 | |
| 6 | 0 | 0 | 0 | 0 | 0 | 0-3 |
| 17 | 0 | 0 | 0-3 | 5 | 15 | |
| 16 | 0 | 0 | 0-3 | 5 | 15 | |
| 29 | 0 | 0 | 0 | 5 | 15 | |
| 9 | 0 | 0 | 0 | 0 | 0 | 0-3 |
| 1 | 0 | 0 | 0 | 0 | 0 | 0-3 |
| 8 | 0 | 0 | 0 | 0 | 0 | 0-3 |
| 19 | 0 | 0 | 3-5 | 5 | 10 | |
| 23 | 0 | 0 | 0 | 0 | 0-3 | 5 |
| 20 | 0 | 0 | 0 | 3-5 | 5 | 10-15 |
| 21 | 0 | 0 | 0 | 3-5 | 5 | 10 |
| 24 | 0 | 0 | 0 | 3 | 5-10 | 15 |
| Vergleichsmittel B | 3 Phytotoxis | 5 Phytotoxis | 10 | 15 | 25 | |
| unbehandelte infiz. Pflanzen | 100 | | | | | |

Beispiel II

Gerstenpflanzen werden im 3-Blattstadium mit Konidien des Gerstenmehltaus (Erysiphe graminis sp. hordei) stark inokuliert und in einem Gewächshaus bei 20°C und einer relativen Luftfeuchte von 90-95% aufgestellt. 3 Tage nach Inokulation werden die Pflanzen mit den in Tabelle II aufgeführten Verbindungen in den Wirkstoffkonzentrationen von 500, 250, 125, 60, 30, 15 und 7,5 mg/Liter Spritzbrühe tropfnaß gespritzt. Als Vergleich wird das Vergleichsmittel B in analoger Weise eingesetzt. Nach einer Inkubationszeit von 10 Tagen werden die Pflanzen auf Befall mit Gerstenmehltau untersucht. Der Befallsgrad wird ausgedrückt in % befallener Blattfäche, bezogen auf unbehandelte,

infizierte Kontrollpflanzen (= 100% Befall). Das Ergebnis ist in der Tabelle II zusammengefaßt.

Tabelle II

| Verbindung gemäß Beispiel Nr. | mit Gerstenmehltau befallene Blattfäche in% bei mg Wirkstoff/Liter Spritzbrühe | | | | | | |
|---|---|---|---|---|---|---|---|
| | 500 | 250 | 125 | 60 | 30 | 15 | 7,5 |
| 15 | 0 | 0 | 3-5 | 5 | 15 | | |
| 6 | 0 | 0 | 0 | 0 | 0 | 0 | 0-3 |
| 17 | 0 | 0 | 0 | 3-5 | 5 | 10-15 | |
| 16 | 0 | 0 | 0 | 5 | 10 | 15 | |
| 30 | 0 | 0 | 3-5 | 5 | 15 | | |
| 29 | 0 | 0 | 0 | 0 | 0-3 | 5 | |
| 9 | 0 | 0 | 0 | 0 | 0 | 0-3 | |
| 1 | 0 | 0 | 0 | 0 | 0 | 0-3 | |
| 8 | 0 | 0 | 0 | 0 | 0 | 0-3 | |
| 19 | 0 | 0 | 0 | 0 | 3 | 5 | |
| 23 | 0 | 0 | 0 | 0 | 0-3 | 3 | |
| 20 | 0 | 0 | 0 | 3-5 | 5-10 | 15 | |
| 21 | 0 | 0 | 0 | 3-5 | 5-10 | 15 | |
| 24 | 0 | 0 | 0 | 0 | 0-3 | 3 | |
| Vergleichsmittel B | 3 | 3-5 | 5 | 5-10 | 15 | 25 | |
| unbehandelte infiz. Pflanzen | 100 | | | | | | |

Beispiel III

Gurkenpflanzen (Sorte Delikateß) werden im 2-Blattstadium mit einer Konidiensuspension von Gurkenmehltau (Erysiphe cichoracearum) stark inokuliert. Nach einer Antrocknungszeit der Sporensuspension von 30 Minuten werden die Pflanzen in einem Gewächshaus bei 22°C und 90% relativer Luftfeuchte aufgestellt. 3 Tage nach Infektion werden die Pflanzen mit den in Tabelle III genannten Verbindungen und Wirkstoffkonzentra-

tionen tropfnaß gespritzt. Als Vergleich wird das Vergleichsmittel A in analoger Weise eingesetzt. Nach 10 Tagen erfolgt die Bonitur. Der Befallsgrad wird ausgedrückt in % befallener Blattfläche, bezogen auf unbehandelte, infizierte Kontrollpflanzen (= 100% Befall). Das Ergebnis ist in der Tabelle III zusammengefaßt.

Tabelle III

| Verbindung gemäß Beispiel Nr. | mit Gurkenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 15 | 0 | 0 | 3-5 | 5 | 15 | |
| 6 | 0 | 0 | 0 | 0 | 0 | 0-3 |
| 17 | 0 | 0 | 0 | 0 | 3 | 5 |
| 16 | 0 | 0 | 0 | 3-5 | 5 | |
| 30 | 0 | 0-3 | 5 | 10 | 15 | |
| 29 | 0 | 0 | 0 | 0 | 0 | 0-3 |
| 9 | 0 | 0 | 0 | 0 | 0 | 0-3 |
| 1 | 0 | 0 | 0 | 0 | 0 | 0-3 |
| 8 | 0 | 0 | 0 | 0 | 0 | 0-3 |
| 19 | 0 | 0 | 0 | 0 | 0-3 | 3 |
| 23 | 0 | 0 | 0-3 | 5 | 10 | 15 |
| 21 | 0 | 0 | 3-5 | 10 | 15 | |
| 24 | 0 | 0 | 5 | 5-10 | 10-15 | |
| Vergleichsmittel A | 3 | 5 | 10 | 15 | 25 | 60 |
| unbehandelte, infiz. Pflanzen | 100 | | | | | |

0015502

Beispiel IV

Gurkenpflanzen (Sorte Delikateß) werden im 2-Blattstadium mit einer Konidiensuspension eines Benomyl-resistenten Gurkenmehltaustammes (Erysiphe cichoracearum) stark inokuliert. Nach einer Antrocknungszeit der Sporensuspension von 30 Minuten werden die Pflanzen in einem Gewächshaus bei 22°C und 90% relativer Luftfeuchte aufgestellt. 3 Tage nach Infektion werden die Pflanzen mit den in Tabelle IV genannten Verbindungen und Wirkstoffkonzentrationen tropfnaß gespritzt. Als Vergleich wird das Vergleichsmittel A in analoger Weise eingesetzt. Nach 10 Tagen erfolgt die Bonitur. Der Befallsgrad wird ausgedrückt in % befallener Blattfäche, bezogen auf unbehandelte, infizierte Kontrollpflanzen (= 100 % Befall). Das Ergebnis ist in der Tabelle IV zusammengefaßt.

Tabelle IV

| Verbindung gemäß Beispiel Nr. | mit Gurkenmehltau (Benomyl-resistenter Stamm) befallene Blattfläche in % bei mg Wirkstoff/ Liter Spritzbrühe | | | | |
|---|---|---|---|---|---|
| | 500 | 250 | 125 | 60 | 30 |
| 15 | 0 | 0 | 3-5 | 5-10 | 15 |
| 6 | 0 | 0 | 0 | 0 | 0-3 |
| 17 | 0 | 0 | 0 | 0 | 5 |
| 16 | 0 | 0 | 3 | 5 | 10 |
| 30 | 0 | 3 | 5 | 10 | 15 |
| 29 | 0 | 0 | 0 | 0 | 0 |
| Vergleichsmittel A | 35 | 60 | 100 | 100 | 100 |
| unbehandelte, infiz. Pflanzen | | | 100 | | |

0015502

Beispiel V

Apfelunterlagen der Sorte EM IX werden im 4-Blattstadium mit einer Konidiensuspension von Apfelmehltau (Podosphaera leucotricha) stark infiziert. Anschließend kommen die Pflanzen für 16 Stunden in eine Klimakammer mit 20°C und einer relativen Luftfeuchte von ca. 100%. Danach werden die Pflanzen in einem Gewächshaus bei 22°C und einer relativen Luftfeuchte von 85% aufgestellt. 3 Tage nach Infektion werden die Pflanzen mit den in Tabelle V genannten Verbindungen und Wirkstoffkonzentrationen tropfnaß gespritzt. Als Vergleich wird das Vergleichsmittel C in analoger Weise eingesetzt. Nach 2-3 Wochen wird der Mehltaubefall bonitiert und der Befallsgrad ausgedrückt in % befallener Blattfläche, bezogen auf unbehandelte, infizierte Kontrollpflanzen (= 100% Befall). Das Ergebnis ist in der Tabelle V zusammengefaßt.

Tabelle V

| Verbindung gemäß Beispiel Nr. | mit Apfelmehltau befallene Blattfläche in % bei mg Wirkstoff/Liter Spritzbrühe | | | | |
|---|---|---|---|---|---|
| | 500 | 250 | 125 | 60 | 30 |
| 15 | 0 | 0-3 | 3-5 | 5 | 10 |
| 6 | 0 | 0 | 0 | 3 | 3-5 |
| 17 | 0 | 0 | 0 | 3 | 5 |
| 16 | 0 | 0-3 | 5 | 5-10 | 10 |
| 30 | 0 | 3 | 5 | 10 | 15 |
| 29 | 0 | 0 | 0 | 0-3 | 3 |
| Vergleichsmittel C | 3 | 5 | 10 | 10-15 | 25 |
| unbehandelte infiz. Pflanzen | 100 | | | | |

0015502
HOE 79/F 048

Patentansprüche:

1. Verbindungen der allgemeinen Formel I

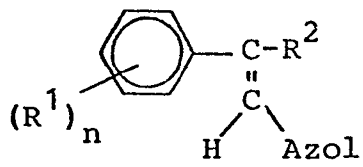

(I)

worin

$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,

n 0, 1, 2 oder 3,

$R^2$ eine $R^3X-\overset{O}{\overset{\|}{C}}$-Gruppe, in der X Sauerstoff oder Schwefel, $R^3$ $C_{1-8}$-Alkyl, das gegebenenfalls durch Halogen, $C_{1-3}$-Alkoxyreste oder durch Alkoxycarbonylreste mit bis zu 5 C-Atomen, oder durch Dialkylaminoreste mit bis zu 8 C-Atomen substituiert ist, $C_{2-6}$-Alkenyl, $C_{2-6}$-Alkinyl, $C_{3-8}$-Cycloalkyl, das gegebenenfalls durch $C_{1-4}$-Alkylreste substituiert ist, Phenyl, das gegebenenfalls durch Halogenatome, $C_{1-3}$-Alkylreste, $C_{1-3}$-Alkoxyreste oder die $CF_3$-Gruppe substituiert ist, Benzyl, das gegebenenfalls durch Halogen substituiert ist, oder einen den Epoxiring enthaltenden Alkylrest mit bis zu 4 C-Atomen bedeutet, oder

$R^2$ für -CN oder eine ${}^{R^4}_{R^5}\!>N-\overset{O}{\overset{\|}{C}}$-Gruppe, in der für $R^4$ Wasserstoff oder $C_{1-4}$-Alkyl und für $R^5$ $C_{1-4}$-Alkyl oder Phenyl, das ggf. durch Halogenatome, $CF_3$-Reste, $C_{1-4}$-Alkyl- oder $C_{1-4}$-Alkoxyreste oder durch den Phenoxy- oder Halogenphenoxyrest substituiert ist, oder $R^4$ und $R^5$ gegebenenfalls gemeinsam mit dem Amid-N-atom einen heterocyclischen Ring bilden, steht,

und

Azol 1,2,4-Triazol-1-yl 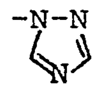 oder

1,2,4-Triazol-4-yl 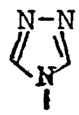 oder

Imidazol-1-yl 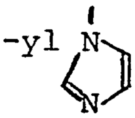

bedeuten. Die Verbindungen der Formel I können auch in Form ihrer Salze mit anorganischen oder organischen Säuren oder als Metallkomplexverbindungen vorliegen.

2. Verfahren zur Herstellung von Verbindungen der allgemeinen Formel I gemäß Anspruch 1, dadurch gekennzeichnet, daß man Verbindungen der allgemeinen Formel II

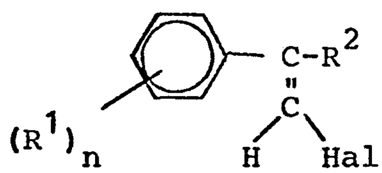

(II)

in der $R^1$, $R^2$ und n die Bedeutungen wie in Formel I haben und Hal für Halogen steht, bei höheren Temperaturen in Gegenwart eines Halogenwasserstoff bindenden Mittels mit mindestens einem Mol 1,2,4-Triazol oder Imidazol pro Mol Verbindung der Formel II, gegebenenfalls in inerten organischen Lösungsmitteln, umsetzt.

3. Schädlingsbekämpfungsmittel, gekennzeichnet durch einen Gehalt an einer Verbindung der Formel I gemäß Anspruch 1 als Wirkstoff.

4. Schädlingsbekämpfungsmittel, gekennzeichnet durch einen Gehalt von 2 bis 95 Gew.-% an einer Verbindung der Formel I gemäß Anspruch 1 als Wirkstoff sowie üblichen Formulierungshilfsmitteln.

5. Verwendung von Verbindungen der Formel I gemäß den Ansprüchen 1, 3, und 4 zur Schädlingsbekämpfung im Pflanzenschutz.

6. Verfahren zur Bekämpfung von Schadpilzen, dadurch gekennzeichnet, daß man die von ihnen befallenen Flächen und/oder Pflanzen und/oder Substrate mit einer fungizid wirksamen Menge von Wirkstoffen der Formel I gemäß Ansprüchen 1, 3 und 4 in Kontakt bringt.

Patentansprüche für Österreich:

1. Verfahren zur Herstellung von Verbindungen der allgemeinen Formel I

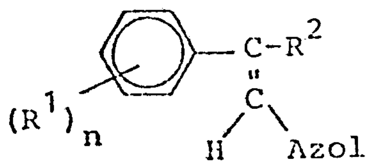

(I)

worin

$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,

n 0, 1, 2 oder 3,

$R^2$ eine $R^3X-\overset{O}{\overset{\|}{C}}-$Gruppe, in der X Sauerstoff oder Schwefel, $R^3$ $C_{1-8}$-Alkyl, das gegebenenfalls durch Halogen, $C_{1-3}$-Alkoxyreste oder durch Alkoxycarbonylreste mit bis zu 5 C-Atomen, oder durch Dialkylaminoreste mit bis zu 8 C-Atomen substituiert ist, $C_{2-6}$-Alkenyl, $C_{2-6}$-Alkinyl, $C_{3-8}$-Cycloalkyl, das gegebenenfalls durch $C_{1-4}$-Alkylreste substituiert ist, Phenyl, das gegebenenfalls durch Halogenatome, $C_{1-3}$-Alkylreste, $C_{1-3}$-Alkoxyreste oder die $CF_3$-Gruppe substituiert ist, Benzyl, das gegebenenfalls durch Halogen substituiert ist, oder einen den Epoxiring enthaltenden Alkylrest mit bis zu 4 C-Atomen bedeutet, oder

$R^2$ für -CN oder eine $\genfrac{}{}{0pt}{}{R^4}{R^5}\!\!>N-\overset{O}{\overset{\|}{C}}-$Gruppe, in der für $R^4$ Wasserstoff oder $C_{1-4}$-Alkyl und für $R^5$ $C_{1-4}$-Alkyl oder Phenyl, das ggf. durch Halogenatome, $CF_3$-Reste, $C_{1-4}$-Alkyl- oder $C_{1-4}$-Alkoxyreste oder durch den Phenoxy- oder Halogenphenoxyrest substituiert ist, oder $R^4$ und $R^5$ gegebenenfalls gemeinsam mit dem Amid-N-atom einen heterocyclischen Ring bilden, steht,

und

Azol 1,2,4-Triazol-1-yl 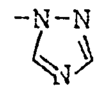 oder

1,2,4-Triazol-4-yl 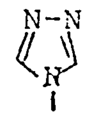 oder

Imidazol-1-yl 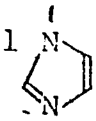

bedeuten, wobei die Verbindungen der Formel I auch in die Form ihrer Salze mit anorganischen oder organischen Säuren oder in Metallkomplexverbindungen überführt werden können, dadurch gekennzeichnet, daß man Verbindungen der allgemeinen Formel II

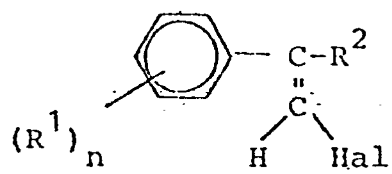 (II)

in der $R^1$, $R^2$ und n die Bedeutungen wie in Formel I haben und Hal für Halogen steht, bei höheren Temperaturen in Gegenwart eines Halogenwasserstoff bindenden Mittels mit mindestens einem Mol 1,2,4-Triazol oder Imidazol pro Mol Verbindung der Formel II, gegebenenfalls in inerten organischen Lösungsmitteln, zu den Verbindungen der Formel I umsetzt und diese gegebenenfalls in Salze mit anorganischen oder organischen Säuren oder in Metallkomplexverbindungen überführt.

2. Schädlingsbekämpfungsmittel, gekennzeichnet durch einen Gehalt an einer Verbindung der Formel I gemäß Anspruch 1 als Wirkstoff.

3. Schädlingsbekämpfungsmittel, gekennzeichnet durch einen Gehalt von 2 bis 95 Gew.-% an einer Verbindung der Formel I gemäß Anspruch 1 als Wirkstoff sowie üblichen Formulierungshilfsmitteln.

4. Verwendung von Verbindungen der Formel I gemäß den Ansprüchen 1, 2 und 3 zur Schädlingsbekämpfung im Pflanzenschutz.

5. Verfahren zur Bekämpfung von Schadpilzen, dadurch gekennzeichnet, daß man die von ihnen befallenen Flächen und/oder Pflanzen und/oder Substrate mit einer fungizid wirksamen Menge von Wirkstoffen der Formel I gemäß Ansprüchen 1, 2 und 3 in Kontakt bringt.